(19) **Europäisches Patentamt**
**European Patent Office**
**Office européen des brevets**

(11) **EP 3 133 928 B1**

(12) **EUROPEAN PATENT SPECIFICATION**

(45) Date of publication and mention
of the grant of the patent:
**27.11.2019 Bulletin 2019/48**

(51) Int Cl.:
*A23B 4/22* *(2006.01)*          *C12R 1/46* *(2006.01)*
*A23L 13/40* *(2016.01)*         *A23L 13/70* *(2016.01)*
*A23B 4/12* *(2006.01)*

(21) Application number: **15710509.9**

(22) Date of filing: **18.03.2015**

(86) International application number:
**PCT/EP2015/055682**

(87) International publication number:
**WO 2015/140211 (24.09.2015 Gazette 2015/38)**

(54) **IMPROVEMENT IN COOKED MEAT MANUFACTURING**

VERBESSERUNG BEI DER HERSTELLUNG VON GEKOCHTEM FLEISCH

AMÉLIORATION DE LA FABRICATION DE VIANDE CUITE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.03.2014 EP 14160754**

(43) Date of publication of application:
**01.03.2017 Bulletin 2017/09**

(73) Proprietor: **DuPont Nutrition Biosciences ApS
1411 Copenhagen K (DK)**

(72) Inventors:
• **FOURCASSIE, Pascal**
  **F-86000 Poitiers (FR)**

• **VOGEL, Christian**
  **CH-8264 Eschenz (CH)**

(74) Representative: **DuPont EMEA
Langebrogade 1
1411 Copenhagen K (DK)**

(56) References cited:
**EP-A1- 2 292 731          WO-A1-97/28702
WO-A1-99/21438             WO-A1-99/47007
WO-A1-2015/007791          GB-A- 1 388 507**

• **BAWA AS, DELONG RO: "Preparation of
Lebanon-Bologna type product using a yoghurt
starter culture", JOURNAL OF FOOD SCIENCE
AND TECHNOLOGY (INDIA), vol. 22, August 1985
(1985-08), pages 262-265, XP009178813,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention relates to the use of lactic acid bacteria strains and compositions comprising these strains for manufacturing cooked meat with improved properties. These strains and compositions are particularly suitable for manufacture of cooked ham, frankfurter sausages and mortadella.

**BACKGROUND OF THE INVENTION**

In cooked meat technology, additives are extensively used to contribute to water retention in order to increase productivity, which can be measured by weight loss of the meat.

Additives commonly used in the meat industry to decrease the weight loss of the meat after cooking encompass phosphates, hydrocolloids (such as guar, xanthane and/or carob) and proteins. However, these additives are not presently satisfactory. Indeed, these additives are not allowed by definition in some premium cooked meat, such as for example Jambon Supérieur. Moreover, the addition of such additives in meat does not fulfill the desires of consumers to eat more natural food products.

A number of documents concerned with increasing yield of cooked meat has been published:

- WO2011/025794 (Bastian et al.) describes the use of milk mineral complex, isolated from milk or whey using filtration, to increase yield of dried or cooked meats.
- WO99/47007 (Bailet et al.) describes the use of starter cultures, in particular starter cultures comprising a single species of microorganisms or a mixture of two or more species of microorganisms. A preferred starter culture is disclosed, which comprises one or more *Stapylococcus spp.* and or or more *Lactobacillus spp*, such as the one exemplified which consists of *Staphylococcus carnosus, Staphylococcus xylosus and Lactobacillus curvatus.* The application focuses on producing a cooked ham which has a yield of at least 90% as compared to the uncooked meat, or which has a yield which is substantially equivalent to that obtainable through the use of phosphate. The last paragraph of the specification concludes that a Jampon supérieur obtained using the exemplified starter culture exhibited a higher yield on cooking relative to a control ham which was prepared without treatment with the starter culture mixture though no values regarding the yield of the Jambon Supérieur and the control ham are disclosed.

Moreover, it is well known to use micro-organisms in meat products. Micro-organisms have traditionally been used for various functions, such as for preserving meat and slowing the growth of spoilage bacteria, or for improving meat quality or meat flavour. Thus, DK166250 describes a method of producing cooked roast beef (fully-cooked meat), heat-treated at a core temperature of 54-63°C, by addition before heat-treatment of a pure culture or a mixed culture of *Staphylococcus xylosus* 2, *Staphylococcus simulans*, *Pediococcus acidilactici*, *Lactobacillus plantarum*, *Lactobacillus carnis*, *Lactobacillus curvatus* and *Pediococcus pentosaceus.* The addition of the bacterial cultures improves both the shelf life and organoleptic properties of the cooked roast beef.

Lactic acid bacteria are also commonly used in the preparation of fermented meats, such as heat treated shelf stable meats (e.g., summer sausage, Lebanon bologna sausage) or not-heat treated shelf stable meats (e.g., pepperoni, salami). During the manufacture of these fermented (shelf stable) meats, lactic acid bacteria (mainly *Lactobacillus* and *Pediococcus*) are added along with simple sugars (such as dextrose) in order to produce lactic acid during the fermentation step, and thus to decrease the pH of the meat to quickly achieve a pH of 5.2 or less. After the fermentation step, the sausages are optionally heat-treated, and dried. The following documents discussed about fermented meat:

- Bawa et al. (Journal of Food Science and Technology 22: 262-265 July/August 1985) describes the preparation of Lebanon-bologna type using a yoghurt starter cultures consisting of equal quantities of *Streptococcus thermophilus* and *Lactobacillus bulgaricus* (today *Lactobacillus delbrueckii subsp. bulgaricus*) diluted in a homogenized milk base (homo milk base). Commercial starter cultures are used in this type of meat product to reduce the fermentation period. Thus, ground meat has been mixed with yoghurt starter culture previously diluted in homo milk base, with or without salt, with either dextrin or lactose, and a fermentation step of about 16h has been carried out. A short cooking step (5h) was applied, with an internal temperature at the end of cooking of 60°C, to give the product without salt some firmness and to shorten the aging period. This publication shows that in absence of salt (batches 1 to 3), both the control starter culture (Saga™) and the yoghurt culture were able to acidify the meat under a pH below 5, during a manufacturing process comprising a fermentation step. However, in presence of salt (batch 4), the activity of the yoghurt culture was inhibited. Thus, the authors conclude that the yoghurt starter culture was found to have very little or no activity in this type of meat product.
- WO97/28702 describes a method for improving meat quality, by addition of a pure lactic acid bacteria culture, such as *Streptococcus lactis subsp. lactis* or *Streptococcus lactis subsp. cremoris* (today known as *Lactococcus lactis subsp lactis* and *Lactococcus lactis subsp cremoris,* respectively), and maturation of the meat with the added lactic acid bacteria at a low temperature of 3-7°C. Maturation is completed when "*there is a faint lactic acid odour, and the pH of the meat has reached 4-5*".

- WO99/21438 describes the manufacture of a cooked sausage comprising mixing a meat emulsion and a mild yoghurt (previously prepared using a slow working yoghurt culture comprising *Lactobacillus acidophilus, Bifidobacteria* and *Streptococcus thermophilus*), and teaches that it is essential that the pH of the meat/yoghurt mixture stays above 5.5, in order that the meat retains its water binding properties

- Finally, various cultures based on *Lactobacillus sakei, Pediococcus acidilactici* and *Staphylococcus xylosus* have been commercialized or assayed to enhance the organoleptic qualities of meat throughout its shelf life, such as texture stabilization, colour stabilization, flavour stabilization and the delay of the development of certain unwanted microflora, without having a negative impact on cooking yield. Examples of such cultures consists of a) *Lactobacillus sakei, Pediococcus acidilactici* and *Staphylococcus xylosus,* b) *Pediococcus acidilactici, Staphylococcus xylosus II* and *Lactococcus lactis subsp lactis biovar diacetylactis,* c) *Lactobacillus sakei, Pediococcus acidilactici, Staphylococcus xylosus II* and *Lactococcus lactis subsp lactis biovar diacetylactis* and d) *Lactobacillus sakei, Staphylococcus xylosus II* and *Lactococcus lactis subsp laetis biovar diacetylaclis.*

Therefore, there is still a need to find means to improve the yield of heat-treated non-fermented cooked meat, in particular fully-cooked not shelf stable meat. The present inventors have surprisingly found that the cooking yield of a fully-cooked not shelf stable meat prepared according to the present invention may be increased, using a lactic acid bacteria species which is specifically found in a milk matrix. Thus, using a bacterial composition comprising at least a *Streptococcus thermophilus* strain, inventors have shown an increase of the cooking yield of a fully-cooked not shelf stable meat by at least 0.5 percentage points as compared with a fully-cooked not shelf stable meat obtained without bacteria addition.

## DESCRIPTION OF THE FIGURES

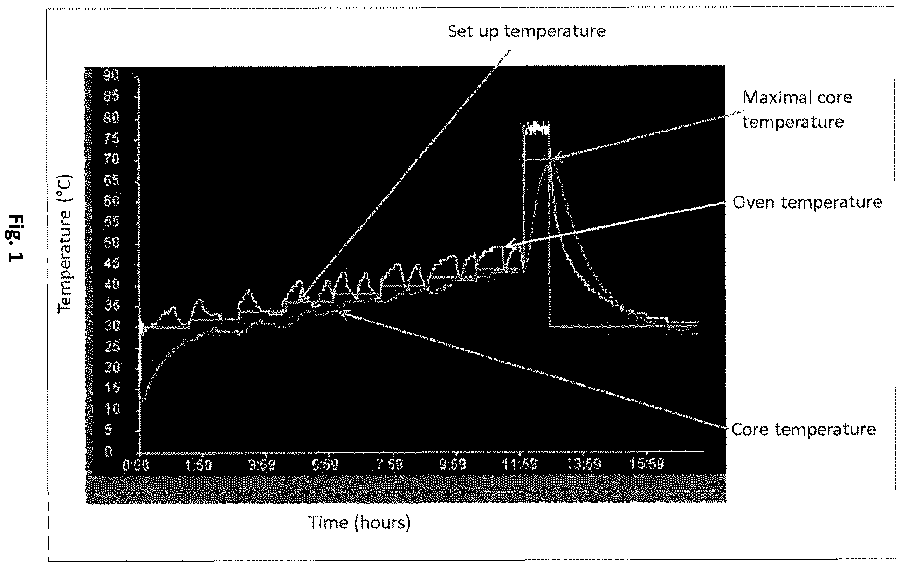

**Figure 1:** graph representing an example of temperature run to obtain a fully-cooked meat. The three curves represent respectively the set-up temperature, the effective temperature in the oven and the core temperature of the meat (all in °C), over time (in hours). In the graph, an arrow also shows the maximal core temperature of the meat (corresponding to the desired core temperature).

## DESCRIPTION OF THE INVENTION

The invention, which is defined by the claims, results from the highlight by the inventors that *Streptococcus thermophilus* strains, strains known for their specificity to dairy environment or dairy matrix, not only can survive in meat matrix, and then can be used in the manufacture of non-fermented heat-treated meats, but also can increase the cooking yield of non-fermented heat-treated meats. *Streptococcus thermophilus* can be used alone or in combination with other microorganisms already found in meat processing, such as *Lactobacillus sakei* strain(s), *Lactobacillus curvatus* strain(s), strain(s) from the *Pediococcus* genus and/or strain(s) from the *Staphylococcus* genus.

In the present application, the expression "*meat*" means the flesh, which is muscle, fat and connective tissue, of mammals used for food, as well as the edible portions of poultry. Meat can be classified as whole-muscle or comminuted meat (*i.e.,* cut or ground meat). In a particular embodiment of any bacterial compositions, methods and uses described herein, meat is the flesh from mammal, in particular from beef and/or from pork. In this embodiment, the meat can be either whole-muscle (for example to manufacture ham or Jambon Supérieur) or comminuted meat (for example to manufacture bologna or frankfurter sausages). Comminuted meat encompasses meat from a mammal or a mixture of meats coming from different mammals, for example comminuted meats from pork and beef. In a particular embodiment of any bacterial compositions, methods and uses described herein, meat is edible portions of poultry, in particular from chicken and/or from turkey. In this embodiment, the meat can be either whole-muscle (for example to manufacture whole boneless turkey breast) or comminuted meat.

The expression "*raw meat*" means a meat before addition of any ingredients which may alter the characteristics of the meat, in particular before marination (addition of functional ingredients such as water, salt, curing agents, phosphates, proteins, carbohydrates, spices). In particular, raw meat refers to meat before addition of the bacterial composition as defined herein.

The expression "*prepared meat*" refers to a meat which has been put into contact with the bacterial composition as defined herein, and which may optionally include, as described in details herein, functional ingredients used for marination and/or a solution such as a marinade, a brine or a cure solution.

The expression "*not shelf stable*" (NSS), refers to meat that needs to be stored/kept refrigerated at or below +5°C or frozen, in particular to maintain safety and quality. This category includes: (a) "*not-fully cooked*" meat, *i.e.,* meat which has received a heat treatment which is not sufficient to result in a ready-to-eat (RTE) meat. Examples of "*not fully cooked NSS*" meat are bacon, and cured and smoke pork product; and (b) "*fully cooked*" meat, *i.e.,* meat which has received a heat treatment, sufficient to result in a RTE meat. Examples of "*fully cooked NSS*" meats are disclosed below. Examples of heat treatment conditions and cooking conditions to obtain a fully cooked meat are defined below.

In the present invention, bacterial composition, methods and use are particularly directed to fully-cooked NSS meat.

In a particular embodiment, the NSS meat, in particular the fully-cooked NSS meat [obtained at the end of the manufacturing process] has a pH value which is above 5.2, is at least 5.3, is at least 5.4, is at least 5.5, is at least 5.6, is at least 5.7 or is at least 5.8. In a particular embodiment, the pH of the meat at any stage of the manufacture of the NSS meat, in particular of the fully-cooked NSS meat [including the pH of the NSS meat or the fully-cooked NSS meat at the end of the manufacturing process] is above 5.2, is at least 5.3, is at least 5.4, is at least 5.5, is at least 5.6, is at least 5.7 or is at least 5.8. In a particular embodiment, the manufacture of the NSS meat, in particular of the fully-cooked NSS meat, is characterized by the absence of fermenting step (*i.e.,* quick and/or important production of lactic acid following the addition of lactic acid bacteria during the process).

In a particular embodiment, the NSS meat, in particular the fully-cooked NSS meat [obtained at the end of the manufacturing process] has a water activity ($a_w$) which is above 0.95, is at least 0.96, is at least or above 0.97, is 0.97 $\pm$ 0.015 or is comprised between 0.955 and 0.98. In a particular embodiment, the water activity ($a_w$) of the NSS meat, in particular the fully-cooked NSS meat, at any stage of the manufacture of the meat [including at the end of the manufacturing process] is above 0.95, is at least 0.96, is at least or above 0.97, is 0.97 $\pm$ 0.015 or is comprised between 0.955 and 0.98. In a particular embodiment, the NSS meat, in particular the fully-cooked NSS meat [obtained at the end of the manufacturing process] has a water activity ($a_w$) which is above 0.91, at least 0.92, at least 0.93, at least 0.94 or at least 0.95, provided that the pH of the meat is at least or above 5.3, is at least or above 5.4, is at least or above 5.5 or is at least or above 5.6. In a particular embodiment, the water activity ($a_w$) of the NSS meat, in particular the fully-cooked NSS meat, at any stage of the manufacture of the meat [including at the end of the manufacturing process] is above 0.91, at least 0.92, at least 0.93, at least 0.94 or at least 0.95, provided that the pH of the meat, at any stage of the manufacture of the meat [including at the end of the manufacturing process], is at least or above 5.3, is at least or above 5.4, is at least or above 5.5 or is at least or above 5.6. In a particular embodiment, the manufacture of the NSS meat, in particular of the fully-cooked NSS meat, is characterized by the absence of drying step (*i.e.*, removal of most of the water present in meat by evaporation or sublimation).

In a particular embodiment, the NSS meat, in particular the fully-cooked NSS meat, has a pH which is above 5.2 and a water activity ($a_w$) which is above 0.95. In a particular embodiment, the NSS meat, in particular the fully-cooked NSS meat, at any stage of the manufacture of the meat [including at the end of the manufacturing process] has a pH which is above 5.2 and a water activity ($a_w$) which is above 0.95. In a particular embodiment, the manufacture of the NSS meat, in particular of the fully-cooked NSS meats, is characterized by the absence of fermenting and drying steps.

In the present invention, bacterial composition, methods and use are particularly directed to fully-cooked NSS meat, in particular fully-cooked NSS meat manufactured from the flesh of mammals used for food or from edible portions of poultry. If necessary, the particular fully-cooked NSS meat described below may be further defined by the pH value and/or the water activity ($a_w$) value described above.

In a particular embodiment, said fully-cooked NSS meat is a cooked deli meat such as, but not limited to, ham, roast beef, pastrami, turkey ham and turkey breast. In a particular embodiment, said fully-cooked NSS meat is a cooked deli meat selected from the group consisting of ham, roast beef, pastrami, turkey ham and turkey breast. In a particular embodiment, the fully-cooked NSS meat is manufactured from the whole muscle of a mammal, in particular from the whole muscle from a beef, and encompasses ham (including Jambon supérieur) and roast beef. In a particular embodiment, the fully-cooked NSS meat is ham. In a particular embodiment, the fully-cooked NSS meat is Jambon supérieur.

In a particular embodiment, the fully-cooked NSS meat is manufactured from comminuted meat from a mammal or mixture of meats coming from different mammals, in particular from beef and/or from pork, and thus encompasses cooked and smoked sausages. In a particular embodiment, the fully-cooked NSS meat is a cooked and smoked sausage such as, but not limited to, bologna, mortadella or frankfurter sausages. In a particular embodiment, said fully-cooked NSS meat is a cooked and smoked sausage selected from the group consisting of bologna, mortadella or frankfurter sausages. In a particular embodiment, the fully-cooked NSS meat is frankfurter sausages.

In a particular embodiment, the fully-cooked NSS meat is manufactured from edible portions of poultry, in particular from chicken and/or from turkey, and thus encompasses or is turkey breast and/or turkey ham.

It is noteworthy that the fully-cooked NSS meat as described herein must be distinguished from the shelf stable meat(s) produced using fermentation and/or produced following the implementation of a significant drying step (*i.e.,* to decrease the $a_w$ of the meat below 0.91). Shelf stable meat(s) are products which can be stored/kept unrefrigerated. These meats are typically characterized by either (a) a pH value below 5.2 and a water activity ($a_w$) of or below 0.95, or (b) a pH value below 5.0, or (c) a water activity ($a_w$) below 0.91. Thus, the fully-cooked NSS meat as described herein is not:

- a non-heated treated shelf stable meat, including dry sausages such as salami or pepperoni; and/or
- a heat-treated shelf stable meat, including semidry sausages such as summer sausages or Lebanon-bologna sausages. It is noteworthy that the heat-treatment and significant drying step of these meats render them safe to store without refrigeration. The heat-treatment is carried out between the fermentation step and the drying step.

It is noteworthy that the fully-cooked NSS meat as described herein is not canned meat, *i.e.,* meat cooked in can, such as spam®.

The expression "*cooking yield*" refers to the ratio of (1) the weight of a meat after heat-treatment to (2) the weight of the same meat before heat-treatment, multiplied by 100 (%). Unless otherwise indicated, meat weight is herein expressed in grams (g). The cooking yield is a consequence of the loss of weight of the meat, as a result of the heat-treatment step.

In the present application, the weight of a meat before heat-treatment means the weight of the meat after the meat has been prepared and before implementing the heat-treatment.

The weight of a meat after heat-treatment means the weight of the meat after it has been cooked and refers in particular to the weight of the NSS meat, in particular to the weight of the fully cooked NSS meat. The weight is determined once the meat is fully cooked (*i.e.,* the cooking is stopped as soon as the desired core temperature of the meat is reached), and no later than one hour after the end of the cooking step, said fully cooked meat being left at room temperature (20-30°C).

For the present invention, a bacterial composition disclosed herein is considered to increase the cooking yield of a fully-cooked NSS meat, when the cooking yield of a fully-cooked NSS meat manufactured using a bacterial composition as disclosed herein is increased by at least 0.5 percentage points as compared to the cooking yield of a control fully-cooked NSS meat. In a particular embodiment, a bacterial composition disclosed herein is considered to increase the cooking yield of a fully-cooked NSS meat, when the average cooking yield of a fully-cooked NSS meat manufactured using a bacterial composition as disclosed herein is increased by at least 0.5 percentage points as compared to the average cooking yield of a control fully-cooked NSS meat, with the average cooking yield defined as the average of the cooking yields of at least 2, and preferably 2, 3 or 4, fully-cooked NSS meats prepared according to the same manufacturing method, at the same time and with the same bacterial composition (or without any bacterial composition for the control), and preferably prepared from the same batch [see examples 3 and 4]. The expression "*cooking yield*" used in the present specification may be replaced by the expression "*average cooking yield*".

A control fully-cooked NSS meat is herein defined as a fully-cooked NSS meat obtained according to the same manufacturing method as the tested fully-cooked NSS, but without any bacterial composition. Therefore, the control fully-cooked NSS meat is manufactured, starting from a meat which does not contain *Streptococcus thermophilus* strain(s) and without addition of a composition comprising bacteria, in particular without addition of a composition of the invention (*i.e.,* without a composition comprising *Streptococcus thermophilus* strain(s)).

It is noteworthy that in order to have a reliable comparison of the effect of the heat treatment on the cooking yield, the weight of the control meat before heat treatment is highly similar to the weight of the tested meat before heat treatment, *i.e.,* the difference between the weight of the control meat before heat treatment and the weight of the tested meat before heat treatment is less than 0.5%.

The increase in cooking yield (I) is calculated as follows:

$$I = \text{cooking yield of tested meat (\%) - cooking yield of a control meat (\%)},$$

and is expressed as percentage points (or pp).

As defined herein, the increase in cooking yield as defined herein (difference between a tested meat and a control meat) or in average cooking yield as defined herein (difference between at least 2 tested meats and at least 2 control meats) is by at least 0.5 percentage points (0.5pp). In a particular embodiment, which can be applied to any bacterial compositions, methods and uses described herein, said increase in cooking yield or said increase in average cooking yield is by at least 0.6pp, at least 0.7pp, at least 0.8pp, at least 0.9pp, at least 1pp, at least 1.5pp, at least 2pp, at least 2.5pp, at least 3pp or at least 3.5pp. In a particular embodiment, which can be applied to any bacterial compositions, methods and uses described herein, said increase in cooking yield or said increase in average cooking yield is by at least 1 percentage points. In a particular embodiment, which can be applied to any bacterial compositions, methods and uses described herein, said increase in cooking yield or said increase in average cooking yield is by at least 1.5 percentage points. In a particular embodiment, which can be applied to any bacterial compositions, methods and uses described herein, said increase in cooking yield or said increase in average cooking yield is by at least 2 percentage points.

The increase in cooking yield as defined herein applies to any fully-cooked NSS meat as defined above.

It is noteworthy that the determination of the weights of a tested meat and the weights of a control meat (before and after heat-treatment) are done at the same time of the manufacturing process.

By "*according to the same manufacturing method*", "*manufactured under the same conditions*" or "*obtained under the same conditions*" which are considered synonymous, it is meant that the tested fully-cooked NSS meat and the control fully-cooked NSS meat are manufactured according to the exact same process (for example, heat treatment, incubation, cooking, inoculation rate ...), with the only exception of the bacterial composition.

The invention relates to a bacterial composition as defined by the claims comprising at least one *Streptococcus thermophilus* strain (which is known to be highly specific for dairy substrate) and at least one strain known and used in meat product manufacturing. Thus, the bacterial composition is suitable for its use in meat product manufacturing.

The invention is directed to a bacterial composition comprising at least one *Streptococcus thermophilus* strain and at least one bacteria selected from the group consisting of a *Lactobacillus sakei* strain, a *Lactobacillus curvatus* strain, a strain from the *Pediococcus* genus and/or a strain from the *Staphylococcus* genus selected from the group consisting of *Staphylococcus vitulinus, Staphylococcus carnosus* and *Staphylococcus xylosus.* In a particular embodiment, said bacterial composition consists of at least one *Streptococcus thermophilus* strain and at least one bacteria selected from the group consisting of a *Lactobacillus sakei* strain, a *Lactobacillus curvatus* strain, a strain from the *Pediococcus* genus and/or a strain from the *Staphylococcus* genus selected from the group consisting of *Staphylococcus vitulinus, Staphylococcus carnosus* and *Staphylococcus xylosus.*

The expression "*at least one*" means one or more, in particular 1, 2, 3, 4, 5 strain(s) or more. In the context of "at least one *Streptococcus thermophilus* strain", this means 1, 2, 3, 4, 5 or more *Streptococcus thermophilus* strain(s). In the context of "*at least one bacteria selected from the group consisting of*", it means one or several bacteria listed in the group, such as for example a *Lactobacillus sakei* strain, a *Lactobacillus curvatus* strain, a strain from the *Pediococcus* genus and a strain from the *Staphylococcus* genus, a *Lactobacillus sakei* strain, a strain from the *Pediococcus genus* and a strain from the *Staphylococcus* genus, a *Lactobacillus sakei* strain, a *Lactobacillus curvatus* strain and a strain from the *Staphylococcus* genus, a *Lactobacillus sakei* strain, a *Lactobacillus curvatus* strain and a strain from the *Pediococcus* genus, a *Lactobacillus curvatus* strain, a strain from the *Pediococcus* genus and a strain from the *Staphylococcus* genus, a *Lactobacillus curvatus* strain and a strain from the *Staphylococcus genus,* a *Lactobacillus curvatus* strain and a strain from the *Pediococcus* genus, and a strain from the *Pediococcus* genus and a strain from the *Staphylococcus* genus.

In a particular embodiment, said bacterial composition comprises or consists of:

- at least one *Streptococcus thermophilus* strain and a *Lactobacillus sakei* strain,
- at least one *Streptococcus thermophilus* strain a *Lactobacillus curvatus* strain,
- at least one *Streptococcus thermophilus* strain and a strain from the *Pediococcus* genus,
- at least one *Streptococcus thermophilus* strain and a strain from the *Staphylococcus* genus selected from the group consisting of *Staphylococcus vitulinus, Staphylococcus carnosus* and *Staphylococcus xylosus.*

As a particular embodiment of any of the bacterial compositions disclosed herein, the bacterial composition is not a composition comprising or consisting of *Lactobacillus acidophilus, Bifodobacteria* and *Streptococcus thermophilus.* In another particular embodiment, of any of the bacterial compositions disclosed herein, the bacterial composition does not comprise a *bifidobacteria.*

As a particular embodiment of any of the bacterial compositions disclosed herein, the bacterial composition does not comprise a *Lactobacillus delbrueckii subsp. bulgaricus* (previously known as *Lactobacillus bulgaricus*) strain. In a particular embodiment, the bacterial composition is not a composition consisting of *Streptococcus thermophilus* and *Lactobacillus delbrueckii subsp. bulgaricus*, and in particular is not a composition consisting of equal quantities of *Streptococcus thermophilus* and *Lactobacillus delbrueckii subsp. bulgaricus.*

In a particular embodiment, said bacterial composition is any of the bacterial compositions described herein, further comprising at least one lactic acid bacteria which in particular is not a *Lactobacillus delbrueckii subsp. bulgaricus* strain. In a particular embodiment, said bacterial composition is any of the bacterial compositions described herein, further comprising at least one lactic acid bacteria which in particular is not a *Bifidobacteria* strain. In a particular embodiment, said bacterial composition is any of the bacterial compositions described herein, further comprising at least one lactic acid bacteria which in particular is neither a *Lactobacillus delbrueckii subsp. bulgaricus* strain nor a *Bifidobacteria* strain.

In a particular embodiment, the bacterial composition comprises or consists of at least one *Streptococcus thermophilus* strain and a *Staphylococcus* strain selected from the group consisting of *Staphylococcus vitulinus, Staphylococcus carnosus* and *Staphylococcus xylosus,* and optionally at least one lactic acid bacteria which in particular is not a *Lactobacillus delbrueckii subsp. bulgaricus* strain and/or is not a *Bifidobacteria* strain.

In a particular embodiment, a strain from the *Staphylococcus* genus is a *Staphylococcus vitulinus* strain, in particular the *Staphylococcus vitulinus* deposited under the Budapest Treaty on January 14, 2014 in the name of Danisco Deutschland GmbH at Leibniz-Institut DSMZ under number DSM28254 [herein the DSM28254 strain].

In a particular embodiment of any of the bacterial compositions defined herein, a strain from the *Pediococcus* genus is a strain selected from the group consisting of a *Pediococcus pentosaceus* and *Pediococcus acidilactici*. In a particular embodiment, a strain from the *Pediococcus* genus is a *Pediococcus pentosaceus* strain, in particular the *Pediococcus pentosaceus* strain deposited under the Budapest Treaty on January 14, 2014 in the name of Danisco Deutschland GmbH at Leibniz-Institut DSMZ under number DSM28253 [herein the DSM28253 strain]. In a particular embodiment, a strain from the *Pediococcus* genus is a *Pediococcus acidilactici strain,* in particular the *Pediococcus acidilactici* strain deposited under the Budapest Treaty on December 9, 2008 in the name of Danisco France SAS at the Collection Nationale de Cultures de Microorganismes under number CNCM 1-4098 [herein the CNCM 1-4098 strain].

In a particular embodiment of any of the bacterial compositions defined herein, said *Lactobacillus sakei* strain is the *Lactobacillus sakei* strain deposited under the Budapest Treaty on January 14, 2014 in the name of Danisco Deutschland GmbH at Leibniz-Institut DSMZ under number DSM28252 [herein the DSM28252 strain].

In a particular embodiment of any of the bacterial compositions defined herein, said bacterial composition, in addition to be defined by the bacteria(s) and possibly microorganism(s) it contains, is further characterized by its ability to increase the cooking yield of a fully-cooked NSS meat by at least 0.5 percentage points as compared to the cooking yield of a control fully-cooked NSS meat obtained under the same conditions but without any bacterial composition. Thus, the bacterial composition, when put into contact with an uncooked (raw) meat, is able to increase or increases the cooking yield of the fully-cooked NSS meat, after this meat being heat-treated up to a core temperature of at least 60°C, by at least 0.5 percentage points as compared to the cooking yield of a fully-cooked NSS meat obtained under the same conditions without any bacterial composition.

The ability of a bacterial composition defined herein to increase the cooking yield of a fully-cooked NSS meat can be checked by the person skilled in the art, in particular implementing the following manufacture process, which is disclosed in more details below:

> 1) putting a raw meat into contact with at least said bacterial composition, to obtain a prepared meat; and
> 2) heat-treating said prepared meat under appropriate conditions to obtain a fully-cooked NSS meat, wherein said heat-treatment comprises cooking said prepared meat up to a core temperature of at least 60°C.

In a particular embodiment, the ability of a bacterial composition defined herein to increase the cooking yield of a fully-cooked NSS meat may be checked implementing the method exemplified in assay I (see example 3 below). All the definitions, disclosed in the context of the method to manufacture a fully-cooked NSS meat below, apply herein, to check this ability.

In a particular embodiment, the *Streptococcus thermophilus* strain or at least one of the *Streptococcus thermophilus* strains of the bacterial composition is salt-tolerant, *i.e.,* is highly resistant to a medium enriched in salt, such as a brine. By "*salt-tolerant*", it is meant that the loss of population of the *Streptococcus thermophilus* strain or of at least one of the *Streptococcus thermophilus* strains of the bacterial composition, does not exceed 3 decimal log, after 24h in a medium enriched in salt, preferably after 24h in a medium enriched in salt at 4°C. In a particular embodiment, the lost does not exceed 2 decimal log, particularly does not exceed 1.5 decimal log, more particularly does not exceed 1 decimal log, after 24h in a medium enriched in salt, preferably after 24h in a medium enriched in salt at 4°C. The tolerance of the *Streptococcus thermophilus* strain or at least one of the *Streptococcus thermophilus* strains of the bacterial composition to salt may be checked as follows:

> (1) inoculating a brine, with a bacterial composition of the invention at a concentration of $1.10^7$ cfu/ml;
> (2) incubating said inoculated medium at 4°C;
> (3) determining the strain concentration by conventional means (for example CFU counting on agar plate), after 15 minutes and after 24h; and
> (4) calculating the loss of strains between the counting at 24h and the counting at 15 minutes.

In a particular embodiment, the tolerance of the *Streptococcus thermophilus* strain or at least one of the *Streptococcus thermophilus* strains of the bacterial composition to salt is determined according to assay II (see example 1 below).

In a particular embodiment, alone or in combination with the salt-tolerant feature, the *Streptococcus thermophilus* strain or at least one of the *Streptococcus thermophilus* strains of the bacterial composition is a low acidifier strain in presence of carbohydrate(s), in particular is a low acidifier strain in presence of sucrose. By "*low acidifier*", it is meant that the *Streptococcus thermophilus* strain or at least one of the *Streptococcus thermophilus* strains of the bacterial composition does not acidify or slightly acidify a medium containing carbohydrate(s), in particular containing sucrose, *i.e.,* that the pH delta of a medium containing carbohydrate(s), in particular containing sucrose, inoculated with a *Streptococcus thermophilus* strain or at least one of the *Streptococcus thermophilus* strains of the bacterial composition is less than 0.25 unit after 21h, in particular after 21h at 45°C [pH delta refers to the difference of the pH of the medium before strain(s) inoculation and the pH of the medium 21h after strain(s) inoculation]. In a particular embodiment, the medium comprises or consists of meat extracts, carbohydrates(s) in particular sucrose, nitrite curing salts and sterile demineralised water. In a particular embodiment, in said medium, the following concentrations may be used:

- meat extract, at a concentration of 150 to 250 g/L of medium, in particular 200 g/L,
- carbohydrate(s), in particular sucrose, at a concentration of 5 to 20 g/L of medium, in particular 10 g/L, and/or
- nitrite curing agents, at a concentration of 10 to 30 g/L of medium, in particular 18 g/L.

In a particular embodiment, said medium is the one disclosed in Table 2 in assay III.

The low acidification ability of the *Streptococcus thermophilus* strain or at least one of the *Streptococcus thermophilus* strains of the bacterial composition may be checked as follows:

(1) inoculating a medium containing carbohydrate(s), in particular containing sucrose, the pH of which is adjusted between 5.84 and 5.86, with a bacterial composition of the invention at a concentration of $6.10^6$ cfu/ml; in a particular embodiment, said medium is as disclosed in Table 2 in assay III;
(2) incubating said inoculated medium at 45°C up to 21 hours in sealed flasks;
(3) measuring the pH of the medium, during the 21 hours; and
(4) calculating the pH variation of the medium between 21 hours and at time of inoculation.

In a particular embodiment, the low acidifying ability of a *Streptococcus thermophilus* strain or at least one of the *Streptococcus thermophilus* strains of the bacterial composition is determined according to assay III (see example 2 below).

In a particular embodiment, the *Streptococcus thermophilus* strain or one of the *Streptococcus thermophilus* strains of the bacterial composition, showing both salt-tolerance feature and low acidifying feature, is a *Streptococcus thermophilus* strain selected from the group consisting of the *Streptococcus thermophilus* strain deposited under the Budapest Treaty on December 4, 2013 in the name of Danisco Deutschland GmbH at Leibniz-Institut DSMZ under number DSM28128 [herein the DSM28128 strain], the *Streptococcus thermophilus* strain deposited under the Budapest Treaty on January 14, 2014 in the name of Danisco Deutschland GmbH at Leibniz-Institut DSMZ under number DSM28255 [herein the DSM28255 strain], the *Streptococcus thermophilus* strain deposited under the Budapest Treaty on January 14, 2014 in the name of Danisco Deutschland GmbH at Leibniz-Institut DSMZ under number DSM28256 [herein the DSM28256 strain], the *Streptococcus thermophilus* strain deposited under the Budapest Treaty on January 14, 2014 in the name of Danisco Deutschland GmbH at Leibniz-Institut DSMZ under number DSM28257 [herein the DSM28257 strain], the *Streptococcus thermophilus* strain deposited under the Budapest Treaty on January 14, 2014 in the name of Danisco Deutschland GmbH at Leibniz-Institut DSMZ under number DSM28258 [herein the DSM28258 strain] and any mixture of two, three, four or five of these strains.

Any of the DSM28128 strain, the DSM28255 strain, the DSM28256 strain, the DSM28257 strain and the DSM28258 strain may be used as a positive control regarding salt-tolerance feature and low acidifying feature, in particular when implementing assay II or assay III. Any of the DSM28128 strain, the DSM28255 strain, the DSM28256 strain, the DSM28257 strain and the DSM28258 strain may be used as a positive control regarding salt-tolerance feature and low acidifying feature on sucrose, in particular when implementing assay II or assay III.

The bacterial or LAB composition of the invention, in particular as a starter culture, may be frozen, dried, freeze dried, liquid, solid, in the form of pellets or frozen pellets, or in a powder or dried powder. In a particular embodiment, the bacterial or LAB composition is in the form of frozen pellets or in the form of a dried powder.

Advantageously, when the bacterial composition or starter culture undergoes a freezing step, cryoprotectant(s) and/or stabilizer(s) is/are mixed with the bacterial composition, in particular after harvesting and possibly concentration, before freezing. Thus, in a particular embodiment, the bacterial composition as defined herein further comprises cryoprotectant(s) and/or stabilizer(s). Cryoprotectants or stabilizers include, but are not limited to, glucose, raffinose, sucrose, trehalose, adonitol, starch, maltitol, hydrogenated glucose syrup (*e.g.*, Lycasin®), maltodextrin, glycerol, mannitol, sorbitol, polypropylene glycol, polyethylene glycol, ribitol, alginate, bovine serum albumin, carnitine, citrate, cystein, dextran, gum such as cassia gum, dimethyl sufoxide, sodium glutamate, sodium chloride, calcium carbonate, magnesium hydroxyl carbonate, glycin, betaine, glycogen, hypotaurin, polyvinyl pirrolidine, taurine, and any combination thereof. The expression "*further comprising*" applies here only to cryoprotectant(s) and/or stabilizer(s). Thus, when a bacterial composition is defined herein as consisting of at least one *Streptococcus thermophilus* strain and possibly another microorganism, the fact that this composition further comprises cryoprotectant(s) and/or stabilizer(s) excludes the addition of any other lactic acid bacteria or any other microorganisms. In other terms, the invention is also directed to a bacterial composition as defined herein into which cryoprotectant(s) and/or stabilizer(s) is/are added.

The bacterial composition, with or without cryoprotectant(s) and/or stabilizer(s), in particular as a starter culture, has a concentration which is at least $10^6$, at least $10^7$, at least $10^8$, at least $10^9$, at least $10^{10}$, at least $10^{11}$ or at least $10^{12}$ CFU/g of the bacterial composition or starter culture, in particular which is between $10^7$ to $10^{12}$ CFU/g of the bacterial composition or starter culture. The concentration applies here to the totality of microorganisms present in the bacterial composition, *i.e.,* depending on the bacterial composition, either *Streptococcus thermophilus* strain(s), or *Streptococcus thermophilus* strain(s) and other lactic acid bacteria(s), or *Streptococcus thermophilus* strain and other microorganisms, or *Streptococcus thermophilus* strain(s), other lactic acid bacteria(s) and other microorganisms.

Thus, a "bacterial composition" or "LAB composition" is not a fermented or partially fermented product obtained after inoculation with bacteria, in particular is not a fermented dairy product obtained after inoculation of milk and/or cream with bacteria. In a particular embodiment, a "bacterial composition" or "LAB composition" is not a dairy product, preferably neither a yogurt nor a mild yogurt.

In a particular embodiment, the "bacterial composition" or "LAB composition" of the invention or used in the method of the invention does not comprise ingredients from milk origin, e.g., does not comprise milk protein or lactose. In a particular embodiment, the "bacterial composition" or "LAB composition" of the invention or used in the method of the invention does not comprise lactose.

As mentioned above, it has been surprisingly found by the inventors that *Streptococcus thermophilus* strains can survive in meat matrix, in particular during the manufacture of a cooked meat. The invention also relates to a method to manufacture a fully-cooked NSS meat, comprising:

1) putting a raw meat into contact with a bacterial composition comprising at least one *Streptococcus thermophilus* strain, wherein said bacterial strain does not comprise lactose, to obtain a prepared meat; and
2) heat-treating said prepared meat under appropriate conditions to obtain a fully-cooked NSS meat, wherein said heat-treatment comprises cooking said prepared meat up to a core temperature of at least 60°C. Said method does not comprise a fermenting step.

In addition to survive in a meat matrix during the manufacture of a cooked meat, it has also been surprisingly shown that fully-cooked NSS meat manufactured, using *Streptococcus thermophilus* strain(s), is characterized by a cooking yield which is increased by at least 0.5 percentage points as compared to the cooking yield of a control fully-cooked NSS meat.

Thus, the present disclosure also relates to a method to manufacture a fully-cooked NSS meat, comprising:

1) putting a raw meat into contact with at least a bacterial composition comprising at least one *Streptococcus thermophilus* strain, to obtain a prepared meat; and
2) heat-treating said prepared meat under appropriate conditions to obtain a fully-cooked NSS meat, wherein said heat-treatment comprises cooking said prepared meat up to a core temperature of at least 60°C,

wherein said fully-cooked NSS meat is characterized by a cooking yield which is increased by at least 0.5 percentage points as compared to the cooking yield of a control fully-cooked NSS meat obtained under the same conditions without any bacterial composition.

In step (1) of the methods of the invention, a bacterial composition as described herein, *i.e.,* a bacterial composition comprising at least one *Streptococcus thermophilus* strain, is put into contact with a raw meat.

Thus, any *Streptococcus thermophilus* strain which can be implemented in the manufacture of a fully-cooked NSS meat product can be used.

In a particular embodiment, the *Streptococcus thermophilus* strain or at least one of the *Streptococcus thermophilus* strains of the bacterial composition is salt-tolerant, in particular as defined and detailed above, in particular according to assay II. In a particular embodiment, the *Streptococcus thermophilus* strain or at least one of the *Streptococcus thermophilus* strains of the bacterial composition is a low acidifier strain in presence of carbohydrate(s), in particular is a low acidifier strain in presence of sucrose, in particular as defined and detailed above, in particular according to assay III. In a particular embodiment, the *Streptococcus thermophilus* strain or at least one of the *Streptococcus thermophilus* strains of the bacterial composition is both salt-tolerant and a low acidifier strain in presence of carbohydrate(s), in particular is a low acidifier strain in presence of sucrose, in particular as defined and detailed above, in particular according to assay II and assay III respectively.

In a particular embodiment, the *Streptococcus thermophilus* strain or at least one of the *Streptococcus thermophilus* strains of the bacterial composition is selected, such that the cooking yield of fully-cooked NSS meat manufactured in presence of said *Streptococcus thermophilus* strain(s) is increased by at least 0.5 percentage points as compared to the cooking yield of a control fully-cooked NSS meat obtained under the same conditions without any bacterial composition. In particular, the *Streptococcus thermophilus* strain or at least one of the *Streptococcus thermophilus* strains of the bacterial composition is selected such that the cooking yield of fully-cooked NSS meat manufactured in presence of said *Streptococcus thermophilus* strain(s) is increased by at least 0.5 percentage points as compared to the cooking yield of a control fully-cooked NSS meat obtained under the same conditions without any bacterial composition, according to assay I.

In a particular embodiment, said *Streptococcus thermophilus* strain or said at least one *Streptococcus thermophilus* strains of the bacterial composition is selected from the group consisting of the DSM28128 strain, the DSM28255 strain, the DSM28256 strain, the DSM28257 strain and the DSM28258 strain and any mixture of two, three, four or five of these strains.

In a particular embodiment, a bacterial composition comprising at least one *Streptococcus thermophilus* strain is a bacterial composition of the invention as described herein, and in particular any bacterial composition comprising at least one *Streptococcus thermophilus* strain and at least one strain known and used in meat manufacturing described above, including any bacterial composition comprising or consisting of at least one *Streptococcus thermophilus* strain and at least one bacteria selected from the group consisting of a *Lactobacillus sakei* strain, a *Lactobacillus curvatus* strain, a strain from the *Pediococcus* genus and/or a strain from the *Staphylococcus* genus, as described above, or any combination of strains or microorganisms as described above.

In a particular embodiment of the methods of the invention, a bacterial composition comprising at least one *Streptococcus thermophilus* strain is not a bacterial composition consisting of *Streptococcus thermophilus* and *Lactobacillus bulgaricus*. In another embodiment, when the meat is comminuted meat, a bacterial composition comprising at least one *Streptococcus thermophilus* strain is not a bacterial composition consisting of *Streptococcus thermophilus* and *Lactobacillus bulgaricus*.

In a particular embodiment, a bacterial composition as described herein, *i.e.,* a bacterial composition comprising at least one *Streptococcus thermophilus* strain does not comprise lactose. Thus, in this embodiment, step (1) of the method comprises or consists in putting a raw meat into contact with at least a bacterial composition comprising at least one *Streptococcus thermophilus* strain, wherein said bacterial composition does not comprise lactose, to obtain a prepared meat.

Within the methods of the invention, the meat (raw meat) is the flesh of mammals used for food, or the edible portions of poultry, either as a whole-muscle or comminuted meat. In a particular embodiment, meat is the flesh from mammal, in particular from beef and/or from pork. In this embodiment, the meat can be either whole-muscle (for example to manufacture ham or Jambon Supérieur) or comminuted meat (for example to manufacture bologna or frankfurter sausages). Comminuted meat encompasses meat from a mammal or a mixture of meats coming from different mammals, for example comminuted meats from pork and beef. In a particular embodiment, the meat is whole-muscle and the fully-cooked NSS meat is ham. In a particular embodiment, the meat is whole-muscle and the fully-cooked NSS meat is Jambon Supérieur. In a particular embodiment, meat is edible portions of poultry, in particular from chicken and/or from turkey. In this embodiment, the meat can be either whole-muscle (for example to manufacture whole boneless turkey breast) or comminuted meat.

Putting the bacterial composition into contact with the raw meat can be carried out by any conventional means known in the art, including soaking, massaging, tumbling, injecting, mixing/blending or any combination thereof. Mixing/blending is primarily used with comminuted meat. Soaking, massaging, tumbling or injecting is mainly used for whole muscle meat [Handbook of Meat and Meat Processing (2nd edition - 2012), chapter 27]. In a particular embodiment, when raw meat is comminuted meat, step (1) is carried out by mixing the bacterial composition with the raw meat. In a particular embodiment, when raw meat is a whole muscle, step (1) is carried out by injecting the bacterial composition into the raw meat, optionally followed by tumbling.

The bacterial composition is put into contact with the raw meat either by addition of the bacterial composition at the surface of the meat or added along with a solution (i.e., a liquid medium). In a particular embodiment of step (1), the bacterial composition is put into contact with the raw meat, under the form of solution. This means that the bacterial composition comprising at least one *Streptococcus thermophilus* strain as defined herein is contained in a solution. This solution can be a marinade, a brine or a cure solution. It is noteworthy that said solution must be suitable for the manufacture of the fully-cooked NSS meat. In a particular embodiment, the bacterial composition comprising at least one *Streptococcus thermophilus* strain is contained in a brine.

Whatever the means to put the bacterial composition in contact with the raw meat, the *Streptococcus thermophilus* strain(s) of the bacterial composition is used at the same conventional concentrations as other starter cultures in the field of meat processing. In a particular embodiment, the *Streptococcus thermophilus* strain(s) of the bacterial composition is used at concentration of at least $1.10^6$ cfu/g, least $5.10^6$ cfu/g, at least $1.10^7$ cfu/g or at least $1.10^8$ cfu/g of prepared meat. "*Prepared meat*" must be understood as defined above and refers to the raw meat supplemented/mixed with the bacterial composition comprising at least one *Streptococcus thermophilus* strain, and optionally functional ingredients used for marination and/or a solution such as a marinade, a brine or a cure solution.

It is noteworthy that the bacterial composition used in step (1) can be frozen, dried, freeze dried, liquid, solid, in the form of pellets or frozen pellets, or in a powder or dried powder. For frozen or dried bacterial composition, addition to the raw meat under the form of a solution, such as a brine, is preferred.

"*Brine*" is as commonly defined in the meat processing technology, *i.e.,* a solution of salt in water, in particular suitable for manufacture of meat products. The definition of brine used herein also applies for the salt-tolerance assay described above. In a particular embodiment, brine is a solution whose salt concentration ranges from 1% to 26%, in particular from 3.5 to 26%, in particular from 3.5 to 11%, in particular from 5% to 20%, in particular from 5 to 10%, from 10 to 15% or from 15 to 20%. In a particular embodiment, the brine contains carbohydrates, such as sucrose/saccharose or dextrose. In a particular embodiment, the concentration of carbohydrates, in particular as sucrose or dextrose, ranges from 0.5 to 15%, in particular from 2 to 6%, in particular from 1 to 5%, from 5 to 10% or from 10 to 15%. In a particular embodiment, the brine contains phosphates (between 0.1 and 5%, in particular between 0.5 and 3%). In a particular embodiment, the brine contains sodium ascorbate and/or stabilizer (such as xanthan or carrageenan). In a particular embodiment, the solution (in particular the brine) comprising the bacterial composition as described herein does not comprise phosphates, in particular when it is used for the manufacture of Jambon Supérieur.

It is understood that the composition of the brine may vary according to the mix rate, *i.e.,* the percentage of brine put in contact with the raw meat to obtain the prepared meat (a mix rate of 1% meaning 1 of brine for 100 of raw meat weight/weight). In a particular embodiment, the brine is put into contact with the raw meat at a mix rate between 5 and 200%, in particular between 10% and 100%, in particular between 40% and 60%, in particular between 10% and 20% by weight of the raw meat. Thus, the less the mix rate, the more the percentage of sugar and the more the percentage of salt in the brine to be mixed. In a particular embodiment, the composition of the brine is determined according to the mix rate and such that the concentration of salt and/or sugars in the prepared meat is according to conventional and accepted values in the cooked meat technology. In a particular embodiment, the composition of the brine is determined such that the concentration of salt in the prepared meat is from 1 to 10 %, in particular from 1 to 3%, from 3 to 5% or from 5 to 10%. In a particular embodiment, combined with the previous one or independently of the previous one, the composition of the brine is determined such that the concentration of sugars in the prepared meat is from 0.5% to 5 %, in particular from 0.5 to 1%, from 1 to 2%, from 2% to 3%, or from 3 to 5%.

A, not limiting, example of brine is a solution consisting of water (86.17%), salt (5.13%), sodium ascorbate (0.14%) and saccharose (8.56%). With a mix rate of 54%, the percentages of the ingredients in the prepared meat are as follows: salt (1.8%), sodium ascorbate (0.05%) and saccharose (3%). Another, not limiting, example of brine is a solution consisting of water (78.10%), salt (10.8%), phosphate (1.8%), dextrose (6%), sodium ascorbate (0.3%) and stabilizer (3%). With a mix rate of 20%, the percentages of the ingredients in the prepared meat are as follows: salt (1.8%), phosphate (0.3%), dextrose (1%), sodium ascorbate (0.05%) and stabilizer (0.5%). Another, not limiting, example of brine is a solution consisting of water (59.85%), salt (19.8%), phosphate (3.3%), dextrose (11%), sodium ascorbate (0.55%) and stabilizer (5.5%). With a mix rate of 10%, the percentages of the ingredients in the prepared meat are as follows: salt (1.8%), phosphate (0.3%), dextrose (1%), sodium ascorbate (0.05%) and stabilizer (0.5%). Another, not limiting, example of brine, in particular used for manufacture of cooked ham, is a solution consisting of water (75.2%), salt (18%), carbohydrates (5%), sodium ascorbate (0.3%) and spices and herbs (1.5%).

In a particular embodiment, step (1) consists in putting the raw meat into contact with the bacterial composition comprising at least one *Streptococcus thermophilus* strain contained in a brine, *i.e.,* to put the raw meat into contact with a brine solution containing the bacterial composition comprising at least one *Streptococcus thermophilus* strain. In a particular embodiment, a brine solution containing the bacterial composition is mixed/blended with comminuted meat, in particular wherein the concentration of *Streptococcus thermophilus* strain(s) in the bacterial composition is at least $1.10^6$ cfu/g or at least $1.10^7$ cfu/g of comminuted prepared meat (*i.e.,* the comminuted meat, the bacterial composition, the brine and optionally other ingredients, such as functional ingredients used for marination). In a particular embodiment, a brine solution containing the bacterial composition is injected into a whole muscle meat (preferably ham), in particular wherein the concentration of *Streptococcus thermophilus* strain(s) in the bacterial composition is at least $1.10^6$ cfu/g or at least $1.10^7$ cfu/g of whole muscle prepared meat (*i.e.,* the whole muscle meat, the bacterial composition, the brine and optionally other ingredients, such as functional ingredients used for marination). In a particular embodiment, the brine solution is injected at a mix rate between 10% and 100%, in particular between 40 and 60%, in particular between 10% and 20% by weight of the raw meat. In a particular embodiment, the injection of the brine solution containing the bacterial composition into a whole muscle meat is followed by tumbling. Tumbling is conventionally carried out during 4 and 10 hours, at a temperature below 10°C, and is optionally followed by a resting period (e.g. for about 4 hours).

As a particular embodiment of step (1), and optionally in combination with any of the embodiments described above, lactose is not present in any of the ingredients used in step (1), including but not limited to, the bacterial composition, the brine, the cryoprotectant(s) and the stabilizer(s). Thus, in this embodiment, step(1) of the method comprises or consists in putting a raw meat into contact with at least a bacterial composition comprising at least one *Streptococcus thermophilus* strain, to obtain a prepared meat, wherein none of the ingredients used for this step (1) contains lactose.

Step (2) consists of the heat-treatment of the prepared meat [following step (1)], under appropriate conditions, to obtain a fully-cooked NSS meat. In a particular embodiment, heat-treatment refers to conditions where the core temperature of the prepared meat is at least 25°C or is at least 30°C. "*Core temperature*" means the internal temperature of the meat.

Within the present invention, the heat-treatment comprises (includes) a step where the prepared meat is cooked up to a core temperature of at least 60°C. In a particular embodiment, the prepared meat is cooked up to a core temperature above 60°C. In a particular embodiment, the prepared meat is cooked up to a core temperature of at least 61, at least 62, at least 63, at least 64, at least 65, at least 66, at least 67, at least 68, at least 69, at least 70°C, at least 71°C or at least 72°C. In a particular embodiment, the prepared meat is cooked up to a core temperature of at least 65°C. In a particular embodiment, the prepared meat is cooked up to a core temperature of at least 68°C. In a particular embodiment, the prepared meat is cooked up to a core temperature of at least 70°C.

The time of heat-treatment may vary with at least one of the following parameters: the type of meat, the weight of the meat, the heat-treatment process and the core temperature of the meat to be reached. The time of heat treatment in the present invention is in agreement with what is generally accepted in the meat technology for a specific fully-cooked NSS meat. In a particular embodiment, the time of heat-treatment ranges from 20 minutes to 24 hours depending on the fully-cooked NSS meat. In a particular embodiment, the time of treatment is between 25 and 45 minutes for frankfurter sausages. In a particular embodiment, the time of treatment is between 3 and 24 hours for a cooked ham. In a particular embodiment, the time of treatment is about 1 hour (e.g., between 45 and 75 minutes) per cm of diameter of meat for mortadella. The times of heat-treatment described above for frankfurter sausages, cooked ham and mortadella are given as non-limiting examples; it is not intended to limit the present invention to this heat-treatment time or specific fully-cooked NSS meat.

Any heat-treatment process (cooking process) known in the art may be used to obtain a fully-cooked NSS meat according to the invention. In a particular embodiment, the heat-treatment is carried out at constant temperature, *i.e.,* the external temperature is maintained constant, at a maximum value, from beginning to end of the thermal processing (the cooking is stopped when the desired core temperature of the meat is reached). In a particular embodiment, the heat-treatment is a step by step cooking, *i.e.,* the external temperature is increased in a graded fashion, in various successive stages (for example by a 2°C increase), until the desired core temperature of the meat is reached. In a particular embodiment, the heat-treatment is a Delta T cooking, *i.e.,* the external temperature is increased continuously, in a line with the increase in temperature of the core temperature of the meat until the desired core temperature of the meat is reached. In a particular embodiment, the heat-treatment is selected from the group consisting of constant temperature cooking, step by step cooking and Delta T cooking.

In a particular embodiment, the heat-treatment comprises or consists of two steps: a first step (2a) where the core temperature of the meat is maintained between 20 and 50°C, in particular between 25 and 45 °C, and a second step (2b) where the temperature is rapidly increased in order the core temperature of the meat reaches at least 60°C. Thus, in particular embodiment, the invention also relates to a method to manufacture a cooked meat, in particular a fully-cooked NSS meat, comprising

1) putting a raw meat into contact with at least a bacterial composition comprising at least one *Streptococcus thermophilus* strain, to obtain a prepared meat;

2a) heat-treating said prepared meat, to a core temperature comprised between 20 and 50°C, in particular between 25 and 45 °C, for at least 3 hours; and

2b) heat-treating the meat obtained in step 2a) up to a core temperature of at least 60°C, under appropriate conditions, to obtain a fully-cooked NSS meat.

In a particular embodiment, the prepared meat is heat-treated to a core temperature between 20 to 50°C, in particular to a core temperature between 25 and 45°C, in particular to a core temperature between 30 and 45°C, and in particular to a core temperature between 35 and 45°C. In a particular embodiment, said step is performed during at least 3 hours, in particular at least 5h, in particular at least 10h, and/or at most 15h.

Thus, in a particular embodiment of heat-treatment, the prepared meat is first heat-treated at a core temperature between 20 and 50° C for at least 3 hours (step 2a), and then heat-treated up to a core temperature of at least or above 60°C, at least 65°C, at least 68°C or at least 70°C, for at least 30 minutes (step 2b). In a particular embodiment, the heat-treatment time is comprised between 3h and 24h, in particular between 10 and 22 hours.

A particular, but non-limiting, example of heat treatment run is disclosed below:

| Step | Temperature | Time |
|---|---|---|
| 1 | 30 °C | 90 min |
| 2 | 32 °C | 90 min |
| 3 | 34 °C | 90 min |
| 4 | 36 °C | 90 min |
| 5 | 38 °C | 90 min |
| 6 | 40 °C | 90 min |

(continued)

| Step | Temperature | Time |
|---|---|---|
| 7 | 42 °C | 90 min |
| 8 | 44 °C | 90 min |
| 9 | 78 °C | up to the desired core temperature of the meat [*i.e.*, at least 61, at least 62, at least 63, at least 64, at least 65, at least 66, at least 67, at least 68, at least 69, at least 70, at least 71 or at least 72°C, according to the present invention] |

Conventional heat-treatment technologies are used, such as oven cooking, steaming or boiling. In a particular embodiment of the invention, heat-treatment is done at atmospheric pressure, at a relative humidity of from 80 to 100%, in particular from 90 to 100%, and in particular from 95 to 100%.

Heat-treatment as defined herein must be distinguished from drying which corresponds to the removal of most of the water present in meat by evaporation or sublimation and thus leads to the decrease in the water activity ($a_w$ value). In a particular embodiment, the process of the invention does not comprise or does not include any drying step. In a particular embodiment, the process of the invention comprises a drying step, provided that the $a_w$ value of the meat [at any stage of the manufacturing process], in particular the $a_w$ value of the NSS meat, in particular of the fully-cooked NSS meat, is as defined above, in particular is above 0.91 when the pH of the fully-cooked NSS meat is at least 5.2, or in particular is at least 0.95.

In a particular embodiment of any methods of manufacture of a fully-cooked NSS meat as described herein, no phosphate is added during the manufacture process, in particular no phosphate is added into the solution (in particular the brine) comprising the bacterial composition as described herein. It is noteworthy that Jambon Supérieur is a high quality fully-cooked ham for which it is not allowed to add phosphates during manufacture. Therefore, in a particular embodiment, the invention is directed to a method to manufacture a cooked ham, in particular a cooked Jambon Supérieur, as defined above, wherein no phosphate is added during the manufacture process.

The invention is also directed to the use of a bacterial composition comprising at least one *Streptococcus thermophilus* strain, wherein said bacterial composition does not comprise lactose, to manufacture a fully-cooked NSS meat, wherein said use does not comprise a fermenting step.

The invention also relates to the use of a bacterial composition comprising at least one *Streptococcus thermophilus* strain, to increase by at least 0.5 percentage points the cooking yield of a fully-cooked NSS meat, manufactured by a method comprising:

1) putting a raw meat into contact with a bacterial composition comprising at least one *Streptococcus thermophilus* strain; and
2) heat-treating said prepared meat under appropriate conditions to obtain a fully-cooked NSS meat, wherein said heat-treatment comprises cooking said prepared meat up to a core temperature of at least 60°C,
as compared to the cooking yield of a fully-cooked NSS meat obtained under the same conditions without any bacterial composition.

The definitions detailed above for the *Streptococcus thermophilus* strain as such, the bacterial composition or the method of manufacture apply herein to any term and expression of the uses.

**EXPERIMENTAL**

**STRAINS**

Herein, DGCC numbers are internal references to DuPont collection; DSM/DSMZ numbers and CNCM I numbers are the numbers assigned respectively by the Leibniz-Institut DSMZ-Deutsche Sammlung von Mikroorganismen und Zellkulturen, GmbH (Inhoffenstr. 7B, D-38124 Braunschweig), and the Collection Nationale de Cultures de Microorganismes (Institut Pasteur, 25 rue du Docteur Roux, F-75724 Paris Cedex 15), following deposit under the Budapest Treaty.

As far as the strain deposited under the Budapest Treaty at the Collection Nationale de Cultures de Microorganismes on December 9, 2008 under number CNCM 1-4098 is concerned, we hereby confirm that the depositor France SAS [of 20 rue Brunel, 75017 Paris France] has authorised the Applicant (DuPont Nutrition Biosciences ApS, of Langebrogade 1, DK-1411 Copenhagen K, Denmark) to refer to the deposited biological materials in this application.

As far as the strains deposited under the Budapest Treaty at the Leibniz-Institut DSMZ-Deutsche Sammlung von Mikroorganismen und Zellkulturen, GmbH, on December 4, 2013 under number DSM28128, and on January 14, 2014 under numbers DSM28252, DSM28253, DSM28254, DSM28255, DSM28256, DSM28257 and DSM28258 are concerned, we hereby confirm that the depositor, Danisco Deutschland GmbH (of Busch-Johannsen-Strasse 1, D-25899 Niebüll, Germany) has authorised the Applicant (DuPont Nutrition Biosciences ApS) to refer to the deposited biological materials in this application.

In respect to those designations in which a European Patent is sought, a sample of these deposited microorganisms will be made available until the publication of the mention of the grant of the European patent, only by the issue of such a sample to an expert nominated by the person requesting the sample, and approved either i) by the Applicant and/or ii) by the European Patent Office, whichever applies (Rule 32 EPC).

## EXAMPLE 1

**Salt-tolerance of *Streptococcus thermophilus* strains**

Assay II

- a brine solution (250g) is prepared by mixing in a sterile flask the following ingredients: 49.5g of nitrite curing salt, 1.375g of Na-ascorbate, and 27.5g of dextrose. Sterile demineralised water is added to complete to 250g. After complete dissolution, the brine is sterilized by filtration on filter $0.20\mu m$. Finally, the brine is distributed in sterile tube (10ml per tube) stored in a water bath at +4°C before inoculation.
- the strain is inoculated at a rate of $1.10^7$ cfu/ml $\pm$ 0.5 log. After strain inoculation and 15 min of stabilization, 1 ml of inoculated brine is sampled in order to evaluate the effective inoculation rate for each strain (Cfu/ml, t=15 min). Then, 1 ml of inoculated brine is sampled after 24 hours at +4°C in order to evaluate the strain salt resistance (Cfu/ml, t=24h).

Application of assay II to 17 *Streptococcus thermophilus* strains

Assay II above has been implemented on 17 strains. Results are summarized in Table 1 below:

Table 1: salt-tolerance of 17 *Streptococcus thermophilus* strains as tested by Assay II

| Strain | Cfu/ml, t=15min | Brine resistance at 4 °C | |
|---|---|---|---|
| | | Cfu/ml, t=24 h | Loss (Log) |
| DSM28256 | 1.30E+05 | 6.00E+05 | -0.66 |
| DGCC5500 | 2.90E+05 | 2.80E+05 | 0.02 |
| DGCC757 | 8.40E+06 | 7.40E+06 | 0.06 |
| DGCC854 | 5.80E+06 | 1.20E+05 | 1.68 |
| DGCC3341 | 6.50E+06 | 7.20E+06 | -0.04 |
| DSM28257 | 7.60E+06 | 2.20E+06 | 0.54 |
| DGCC2057 | 3.70E+06 | 4.70E+04 | 1.90 |
| DGCC2062 | 1.20E+07 | 1.80E+05 | 1.82 |
| DGCC7854 | 1.20E+08 | 7.10E+07 | 0.23 |
| DGCC8006 | 1.70E+08 | 1.40E+08 | 0.08 |
| DGCC11229 | 4.40E+06 | 1.80E+04 | 2.39 |
| DSM28258 | 7.70E+06 | 1.20E+06 | 0.81 |
| DGCC7963 | 5.60E+06 | 2.90E+05 | 1.29 |
| DSM28128 | 1.50E+07 | 5.00E+05 | 1.48 |
| DGCC7773 | 1.50E+06 | 3.70E+04 | 1.61 |
| DSM28255 | 1.04E+07 | 3.40E+06 | 0.49 |

(continued)

| Strain | Cfu/ml, t=15min | Brine resistance at 4 °C | |
|---|---|---|---|
| | | Cfu/ml, t=24 h | Loss (Log) |
| DGCC7710 | 1.40E+07 | 9.30E+06 | 0.18 |

The studied *Streptococcus thermophilus* strains are tolerant to salt and thus resistant to osmotic pressure, with 8 strains presenting a loss less than 0.5 log of population, 2 strains presenting a loss comprised between 0.5 log and 1 log, 2 strains presenting a loss comprised between 1 log and 1.5 log, 4 strains presenting a loss comprised between 1.5 log and 2 log, and 1 strain presenting a loss comprised between 2 log and 3 log, after 24 hours at +4°C in brine.

## EXAMPLE 2

**Low acidification of *Streptococcus thermophilus* strains**

Assay III

A liquid meat model medium (LMMM) [medium containing carbohydrate] for the low acidification assay is prepared, with the following composition:

Table 2: Composition of the LMMM medium

| Ingredients | Reference | Weight |
|---|---|---|
| Meat extracts: | - | - |
| - OrganoTechnie (60%) | M1 | 120 g |
| - Difco (20%) | Bacto beef extract, ref 211520 | 40 g |
| - Kerry RL (20%) | 5X00128 | 40g |
| Sucrose | PROLABO, ref. 24-379-296 | 10 g |
| Nitrite curing salt | - | 18 g |
| Antifoam Biospumex 0,5 | - | 4 - 5 drops |
| Sterile demineralised water | - | qsp 1000 ml |

- Media preparation: a part of the sterile demineralised water (previously tempered at 45°C) is added in a non sterile plastic beaker with a magnetic stirrer bar. Sugar (sucrose) and then nitrite curing salt are added and dissolved by magnetic agitation. After they have been dissolved, meat extracts are added one by one. After complete dissolution of meat extracts, antifoam is added drop by drop until foam has disappeared. Sterile demineralised water is added to complete to final volume.
- pH adjustment, dispatching of media and inoculation: a water proof magnetic stirrer is placed in a water bath at 45°C. The beaker containing LMMM is put in the water bath on the magnetic stirrer and agitated during 30 minutes to stabilize temperature. pH of LMMM is adjusted to 5.90 with lactic acid (pH probe previously calibrated). After 30 minutes (for pH stabilization), pH of LMMM is adjusted to 5.84 - 5.86 with lactic acid addition drop by drop. After about 30 minutes (for pH stabilization), LMMM is dispatched in plastic bottles with a graduated cylinder of 50 ml. pH probes are sanitized with ethanol 70% (v/v) and rinsed with sterile demineralised water. Finally, pH probes are placed in plastic bottles.
- each bottle is inoculated with a *Streptococcus thermophilus* strain at a rate of $6.10^6$ cfu/ml, and the bottle is sealed with parafilm. The pH of the inoculated medium after incubation in the water bath at 45°C during 21h (pH, t=21h) is measured. The pH delta [difference of the pH of the medium before strain(s) inoculation and the pH of the medium 21h after strain(s) inoculation] is then calculated.

Application of assay III to 9 *Streptococcus thermophilus* strains

Assay III above has been implemented on 9 strains. Results are summarized in Table 3 below:

Table 3: acidification feature of 9 *Streptococcus thermophilus* strains as tested by Assay III

| Strain | pH, t=0 | pH, t=21h | pH delta |
|---|---|---|---|
| DSM28256 | 5.85 | 5.84 | -0.01 |
| DGCC757 | 5.85 | 5.88 | +0.03 |
| DGCC3341 | 5.85 | 5.84 | -0.01 |
| DSM28257 | 5.86 | 5.88 | +0.02 |
| DGCC7854 | 5.85 | 5.68 | -0.17 |
| DGCC8006 | 5.84 | 5.69 | -0.15 |
| DSM28258 | 5.87 | 5.84 | -0.03 |
| DSM28128 | 5.87 | 5.80 | -0.07 |
| DSM28255 | 5.86 | 5.84 | -0.02 |

From the 9 tested strains, two strains (DGCC7854 and DGCC8006) slightly acidify the LMMM after 21h at 45°C (pH delta of 0.17 unit and 0.15 unit respectively). All the tested strains have a pH delta which is less than 0.25 unit after 21h (by assay III) and are thus considered suitable for used in meat processing.

## EXAMPLE 3

**Increase in the cooking yield of a fully cooked NSS meat using 6 *Streptococcus thermophilus* strains**

Assay I

- a fully cooked NSS meat is prepared following the protocol as detailed hereinafter, with the ingredients in the following final proportions (Table 4):

Table 4: ingredients of a prepared meat according to assay I; †: for tested meat, the *Streptococcus thermophilus* strain(s) is added to the brine, in order the inoculation rate of this/these strain(s) is at least $1.10^6$ cfu / g of prepared meat.

| Ingredients | | Weight (g) | % |
|---|---|---|---|
| Pork shoulder 3% - 6% Fat | | 977.25 | 65.150 |
| Brine | Water | 450.00 | 30.000 |
| | Nitrite Salt 0.6% | 27.00 | 1.800 |
| | Saccharose | 45.00 | 3.000 |
| | Sodium Ascorbate Powder | 0.75 | 0.050 |
| | Total † | 1500.00 | 100.000 |

- brine preparation: the appropriate volume of water is prepared one day before in the fridge to get a temperature between 0 and 5°C. First, the nitrate salt is dissolved for 2 min 1100U/min with a Lab Mixer (RW 20 DZM Janke & Kunkel). Then, the other ingredients (preblended) are dispersed at the end of the process for 1 min. When used, the strain (presolubilized) is added to the brine just before the end of the mixing, in order the inoculation rate of this/these strain(s) is at least $1.10^6$ cfu / g of prepared meat (meat and brine). In a particular embodiment of assay I, the inoculation rate of this/these strain(s) is $1.10^7$ cfu / g of prepared meat.
- meat preparation: the meat (pork shoulder) is grinded through a 3mm plate. The grounded meat is placed in a mixer and 1/2 of the brine is added and mixed for 30 sec and then for 4 min and 30 sec. Then, the bowl is scraped, and the remaining of the brine is added and mixed for 30 sec and then for 4 min and 30 sec. 1 minute before the end of the mixing process, the mixer is stopped and the bowl is scraped again. The prepared meat is left for 20h at 4°C. Each batch (one control batch and one batch for each tested strain) is mixed again for 2 min. Plastic cups are then filed with the prepared meat (standardized weigh of 220 g prepared meat per cup). Cups are sealed with a plastic foil.

- heat-treatment: cups are placed in a steam oven, and the following heating program is implementing (Table 5). Step 9 is applied until the core temperature of meat reaches the desired temperature. Within the present invention, the core temperature of the meat to be reached is at least 60°C, at least 61, at least 62, at least 63, at least 64, at least 65, at least 66, at least 67, at least 68, at least 69, at least 70°C, at least 71°C or at least 72°C. In present assay I, the core temperature to be reached is 70°C. After cooking, cups are left for 1h at room temperature and the cooking yield is calculated.

Table 5: example of temperature run; †: in assay I, the desired core temperature is 70°C.

| Step | Temperature | Time |
|---|---|---|
| 1 | 30 °C | 90 min |
| 2 | 32 °C | 90 min |
| 3 | 34 °C | 90 min |
| 4 | 36 °C | 90 min |
| 5 | 38 °C | 90 min |
| 6 | 40 °C | 90 min |
| 7 | 42 °C | 90 min |
| 8 | 44 °C | 90 min |
| 9 | 78 °C | up to the desired core temperature † |

Application of assay I to 6 *Streptococcus thermophilus* strains

*6 Streptococcus thermophilus* strains have been used in the manufacturing of cooked meat as described in assay I. The inoculation rate is $1.10^7$ cfu/g of prepared meat. For each batch, 4 cups have been prepared and heat-treated (cups 1 to 4 in Table 6 below).

| Step # | Temperature | Time |
|---|---|---|
| Step 1 | 30 °C | 90 min |
| Step 2 | 32 °C | 90 min |
| Step 3 | 34 °C | 90 min |
| Step 4 | 36 °C | 90 min |
| Step 5 | 38 °C | 90 min |
| Step 6 | 40 °C | 90 min |
| Step 7 | 42 °C | 90 min |
| Step 8 | 44 °C | 90 min |
| Step 9 | 78 °C | * |

*: until the core temperature of meat reaches 70°C

The average cooking loss and average cooking yield for these 4 cups have been calculated (CL1 to CL4 and CY1 to CY4 respectively) and are disclosed in Table 6 below. The following abbreviations have been used: BH: weight before heat-treatment; AH: weight after heat-treatment; CL: cooking loss in % [(AH/BH)-1 x 100]; Std. Dev CL: Standard deviation of the CL average; CY: cooking yield (AH/BH); Average CY: average cooking yield. The control batch (no inoculation) is specific to the experiment.

Table 6: cooking yields and average cooking yield of fully cooked NSS meats prepared using 6 *Streptococcus thermophilus* strains as tested by Assay I; †: weight in gram

| | Control | DGCC 7854 | DGCC 8006 | DSM 28255 | DSM 28257 | DSM 28258 | DSM 28128 |
|---|---|---|---|---|---|---|---|
| BH 1 † | 220.76 | 220.96 | 220.52 | 220.74 | 220.88 | 220.26 | 220.46 |
| BH 2 | 220.60 | 220.45 | 220.50 | 220.25 | 220.28 | 220.70 | 220.65 |
| BH 3 | 220.34 | 220.50 | 220.87 | 220.55 | 220.11 | 220.13 | 220.86 |
| BH 4 | 220.55 | 220.71 | 220.16 | 220.00 | 220.20 | 220.88 | 220.68 |
| AH 1 † | 190.61 | 194.18 | 195.82 | 196.71 | 197.10 | 197.59 | 198.67 |
| AH 2 | 188.93 | 194.23 | 195.00 | 196.72 | 196.26 | 198.11 | 198.12 |
| AH 3 | 190.19 | 192.59 | 195.12 | 196.77 | 197.67 | 197.66 | 199.18 |
| AH 4 | 190.57 | 193.37 | 195.32 | 196.95 | 197.00 | 197.49 | 199.25 |
| CL1 (%) | -13.66% | -12.12% | -11.20% | -10.89% | -10.77% | -10.29% | -9.88% |
| CL2 (%) | -14.36% | -11.89% | -11.56% | -10.68% | -10.90% | -10.24% | -10.21% |
| CL3 (%) | -13.68% | -12.66% | -11.66% | -10.78% | -10.19% | -10.21% | -9.82% |
| CL4 (%) | -13.59% | -12.39% | -11.28% | -10.48% | -10.54% | -10.59% | -9.71% |
| Average CL | -13.82% | -12.26% | -11.43% | -10.71% | -10.60% | -10.33% | -9.91 % |
| Std. Dev CL | 0.36% | 0.33% | 0.22% | 0.17% | 0.31% | 0.18% | 0.22% |
| CY1 (%) | 86.34% | 87.88% | 88.80% | 89.11% | 89.23% | 89.71% | 90.12% |
| CY2 (%) | 85.64% | 88.11% | 88.44% | 89.32% | 89.10% | 89.76% | 89.79% |
| CY3 (%) | 86.32% | 87.34% | 88.34% | 89.22% | 89.81% | 89.79% | 90.18% |
| CY4 (%) | 86.41% | 87.61% | 88.72% | 89.52% | 89.46% | 89.41% | 90.29% |
| Average CY | 86.18% | 87.74% | 88.57% | 89.29% | 89.40% | 89.67% | 90.09% |

From the 6 tested strains, one strain (DGCC7854) increases the cooking yield and the average cooking yield of fully-cooked NSS meat(s) by more than 1pp, as compared to the cooking yield and average cooking yield of control meat(s). One strain (DGCC8006) increases the cooking yield and the average cooking yield of fully-cooked NSS meat(s) by more than 2pp, as compared to the cooking yield and average cooking yield of control meat(s). Two strains (DSM28255 and DSM28257) increase the cooking yield of fully-cooked NSS meat by more than 2.5pp, and the average cooking yield of fully-cooked NSS meats by more than 3pp, as compared to the cooking yield and average cooking yield of control meats respectively. One strain (DSM28258) increases the average cooking yield of fully-cooked NSS meats by more than 3pp, as compared to the average cooking yield of control meats. Finally, one strain (DSM28128) increases the average cooking yield of fully-cooked NSS meats by more than 3.5pp, as compared to the average cooking yield of control meats.

The pH of the raw meat, the prepared meat before heat treatment (mean of 4 cups) and the fully cooked NSS meat (mean of 4 cups) has been measured. The measurements are summarized in the following table:

Table 7: pH measured during the manufacture of fully cooked NSS meats prepared using 6 *Streptococcus thermophilus* strains

| pH | Control | DGCC 7854 | DGCC 8006 | DSM 28255 | DSM 28257 | DSM 28258 | DSM 28128 |
|---|---|---|---|---|---|---|---|
| raw meat | 5.78 | 5.71 | 5.79 | 5.84 | 5.84 | 5.73 | 5.79 |
| BH | 5.80 | 5.85 | 5.92 | 5.90 | 5.94 | 5.95 | 5.99 |
| AH | 6.13 | 6.01 | 5.74 | 6.08 | 5.93 | 5.90 | 5.97 |

For all the used *Streptococcus thermophilus* strains, the pH of the meat at the end of the meat processing (fully cooked NSS meat) as well as the pH of the meat during the whole manufacturing process is at least 5.80.

Altogether, these results confirm that a bacterial composition comprising at least *Streptococcus thermophilus* strain(s) may be used during the processing of fully cooked NSS meat, to significantly increase the cooking yield of the fully cooked NSS meat, while maintaining the pH of the meat above 5.2.

**EXAMPLE 4**

**Increase in the cooking yield of a fully cooked NSS meat using the DSM28128 *Streptococcus thermophilus* strain at 2 different inoculation rates**

The DSM28128 *Streptococcus thermophilus* strain has been used in the manufacturing of cooked meat as described in assay I. The inoculation rate is either $1.10^6$ cfu/ g of prepared meat or $1.10^7$ cfu/ g of prepared meat. For each batch, 4 cups have been prepared and heat-treated (cups 1 to 4 in the Table below). The temperature run is as follows and also disclosed in Figure 1.

| Step # | Temperature | Time |
|--------|-------------|------|
| Step 1 | 30 °C | 90 min |
| Step 2 | 32 °C | 90 min |
| Step 3 | 34 °C | 90 min |
| Step 4 | 36 °C | 90 min |
| Step 5 | 38 °C | 90 min |
| Step 6 | 40 °C | 90 min |
| Step 7 | 42 °C | 90 min |
| Step 8 | 44 °C | 90 min |
| Step 9 | 78 °C | * |

*: until the core temperature of meat reaches 70°C

The average cooking loss and average cooking yield for these 4 cups have been calculated and are disclosed in Table 8 below. In table 8, the following abbreviations have been used: BH: weight before heat-treatment; AH: weight after heat-treatment; CL: cooking loss in % [(AH/BH)-1 x 100]; Std. Dev CL: Standard deviation of the CL average; CY: cooking yield (AH/BH); Average CY: average cooking yield. The control batch (no inoculation) is specific to the experiment.

Table 8: cooking yields and average cooking yield of fully cooked NSS meats prepared using the DSM 28128 strain as tested by Assay I; †: weight in gram

|  | Control | DSM28128 | DSM28128 |
|--------|---------|----------|----------|
| Inoculation rate (cfu / g of mixed meat) | - | $1.10^6$ | $1.10^7$ |
| BH 1† | 220.17 | 220.92 | 220.01 |
| BH 2 | 220.80 | 220.24 | 220.44 |
| BH 3 | 220.98 | 220.56 | 220.99 |
| BH 4 | 220.83 | 220.39 | 220.81 |
| AH 1† | 190.71 | 192.92 | 197.09 |
| AH 2 | 191.19 | 193.69 | 196.85 |
| AH 3 | 191.81 | 193.11 | 197.92 |
| AH 4 | 191.57 | 193.63 | 196.71 |
| CL1 (%) | -13.38% | -12.67% | -10.42% |
| CL2 (%) | -13.41% | -12.06% | -10.70% |
| CL3 (%) | -13.20% | -12.45% | -10.44% |
| CL4 (%) | -13.25% | -12.14% | -10.91% |

(continued)

|  | Control | DSM28128 | DSM28128 |
|---|---|---|---|
| Average CL | -13.31% | -12.33% | -10.62% |
| Std. Dev | 0.10% | 0.28% | 0.24% |
| CY1 (%) | 86.62% | 87.33% | 89.58% |
| CY2 (%) | 86.59% | 87.94% | 89.30% |
| CY3 (%) | 86.80% | 87.55% | 89.56% |
| CY4 (%) | 86.75% | 87.86% | 89.09% |
| Average CY | 86.69% | 87.67% | 89.38% |

The DSM28128 strain, when used at an inoculation rate of $1.10^7$ cfu/ g of prepared meat, increases the cooking yield of fully-cooked NSS meat by more than 2.5pp, as compared to the average cooking yield of control meats. The same strain, used at an inoculation rate of $1.10^6$ cfu/ g of prepared meat, still increases the average cooking yield of fully-cooked NSS meats by almost 1pp, as compared to the average cooking yield of control meats.

The pH of the prepared meat before heat treatment (mean of 4 cups) and the fully cooked NSS meat (mean of 4 cups) has been measured. The measurements are summarized in the following table:

Table 9: pH measured during the manufacture of fully cooked NSS meats prepared using the DSM 28128 strain

| pH | Control (-) | DSM28128 ($1.10^6$) | DSM28128 ($1.10^7$) |
|---|---|---|---|
| BH | 5.72 | 5.72 | 5.79 |
| AH | 6.01 | 6.05 | 5.91 |

Using the DSM28128 strain, the pH of the meat at the end of the meat processing (fully cooked NSS meat) as well as the pH of the meat during the whole manufacturing process is at least 5.72.

**Altogether, these results confirm that a bacterial composition comprising at least *Streptococcus thermophilus* strain(s) may be used at an inoculation rate as low as of $1.10^6$ cfu/g of prepared meat, and in particular at an inoculation rate of $1.10^7$ cfu/g of prepared meat, during the processing of fully cooked NSS meat, to significantly increase the cooking yield of the fully cooked NSS meat, while maintaining the pH of the meat above 5.2.**

## Claims

1. Bacterial composition, suitable for the manufacture of fully-cooked not shelf stable (NSS) meat, comprising at least one *Streptococcus thermophilus* strain and at least one bacteria strain selected from the group consisting of a *Lactobacillus sakei* strain, a *Lactobacillus curvatus* strain, a strain from the *Pediococcus* genus selected from the group consisting of *Pediococcus acidilactici* and *Pediococcus pentosaceus* and/or a strain from the *Staphylococcus* genus selected from the group consisting of *Staphylococcus vitulinus, Staphylococcus carnosus* and *Staphylococcus xylosus*.

2. Bacterial composition according to claim 1, wherein said at least one *Streptococcus thermophilus* strain is a low acidifier strain in presence of carbohydrate(s), in particular is a low acidifier strain in presence of sucrose, and/or is highly resistant in brine.

3. Bacterial composition according to claim 1 or 2, comprising at least one *Streptococcus thermophilus* strain and a *Staphylococcus* strain selected from the group consisting of *Staphylococcus vitulinus, Staphylococcus carnosus* and *Staphylococcus xylosus,* and optionally at least one lactic acid bacteria.

4. Bacterial composition according to any one of claims 1 to 3, wherein said *Staphylococcus* strain is a *Staphylococcus vitulinus,* in particular the strain deposited under the Budapest Treaty on January 14, 2014 at Leibniz-Institut DSMZ under number DSM28254.

5. Bacterial composition according to any one of claims 1 to 4, which is frozen, dried, freeze dried, liquid, solid, in the form of pellets or frozen pellets, or in a powder or dried powder.

6. Method to manufacture a fully-cooked NSS meat, comprising:

1) putting a raw meat into contact with at least a bacterial composition comprising at least one *Streptococcus thermophilus* strain, wherein said bacterial composition does not comprise lactose, to obtain a prepared meat; and
2) heat-treating said prepared meat under appropriate conditions to obtain a fully-cooked NSS meat, wherein said heat-treatment comprises cooking said prepared meat up to a core temperature of at least 60°C;

wherein said method does not comprise a fermenting step.

7. Method to manufacture a fully-cooked NSS meat according to claim 6, comprising:

1) putting a raw meat into contact with at least a bacterial composition comprising at least one *Streptococcus thermophilus* strain, to obtain a prepared meat,
2a) heat-treating said prepared meat, to a core temperature comprised between 20 and 50°C, for at least 3 hours; and
2b) heat-treating the meat obtained in step 2a) up to a core temperature of at least 60°C, under appropriate conditions, to obtain a fully-cooked NSS meat.

8. Method to manufacture a fully-cooked NSS meat according to claim 6 or 7, wherein said at least one *Streptococcus thermophilus* strain is a low acidifier strain in presence of carbohydrate(s), in particular is a low acidifier strain in presence of sucrose, and/or is highly resistant in brine.

9. Method according to any one of claims 6 to 8, wherein said bacterial composition is as defined in any one of claims 1 to 5.

10. Method according to any one of claims 6 to 9, wherein said meat is cooked up to a core temperature of at least 65°C, in particular at least 68°C, in particular at least 70°C.

11. Method according to any one of claims 6 to 10, wherein the raw meat is put into contact with said bacterial composition and with a brine, in particular with said bacterial composition contained in a brine.

12. Method according to any one of claims 6 to 11, wherein said bacterial composition is put into contact with the raw meat at a concentration of at least $1.10^6$ CFU / g of the prepared meat, in particular at a concentration of at least $5.10^6$ or at least $1.10^7$ CFU / g of the prepared meat.

13. Bacterial composition according to any one of claims 1 to 5 or method according to any one of claims 6 to 12, wherein said at least one *Streptococcus thermophilus* strain is a low acidifier strain as determined by assay III and/or is a salt-tolerant strain as determined by assay II.

14. Bacterial composition according to any one of claims 1 to 5 and 13 or method according to any one of claims 6 to 13, wherein said *Streptococcus thermophilus* strain or said at least one *Streptococcus thermophilus* strain either is or is selected from the group consisting of the strain deposited at the DSMZ on December 4, 2013 under number DSM28128, the strain deposited at the DSMZ on January 14, 2014 under number DSM28255, the strain deposited at the DSMZ on January 14, 2014 under number DSM28256, the strain deposited at the DSMZ on January 14, 2014 under number DSM28257, the strain deposited at the DSMZ on January 14, 2014 under number DSM28258 and any mixture thereof.

15. Method according to any one of claims 6 to 14, wherein said meat is a whole muscle meat, in particular ham or is chunks of meat.

16. Use of a bacterial composition comprising at least one *Streptococcus thermophilus* strain, wherein said bacterial composition does not comprise lactose, to manufacture a fully-cooked NSS meat, wherein said use does not comprise a fermenting step.

**Patentansprüche**

1. Bakterielle Zusammensetzung, geeignet für die Herstellung von vollständig gekochtem, nicht lagerstabilem (NSS) Fleisch, umfassend mindestens einen Stamm *Streptococcus thermophilus* und mindestens eine bakterielle Zusammensetzung, die ausgewählt ist aus der Gruppe bestehend aus einem Stamm *Lactobacillus sakei,* einem Stamm *Lactobacillus curvatus,* einem Stamm aus der Gattung *Pediococcus,* der ausgewählt ist aus der Gruppe bestehend aus *Pediococcus acicilactici* und *Pediococcus pentosaceus,* und/oder einem Stamm aus der Gattung *Staphylococcus,* der ausgewählt ist aus der Gruppe bestehend aus *Staphylococcus vitulinus*, *Staphylococcus carnosus* und *Staphylococcus xylosus.*

2. Bakterielle Zusammensetzung nach Anspruch 1, wobei der mindestens eine Stamm *Streptococcus thermophilus* ein gering ansäuernder Stamm in Gegenwart von Kohlenhydrat(en) ist, speziell ein gering ansäuernder Stamm in Gegenwart von Saccharose ist und/oder in Salzlösung hoch resistent ist.

3. Bakterielle Zusammensetzung nach Anspruch 1 oder 2, umfassend mindestens einen Stamm *Streptococcus thermophilus* und einen Stamm *Staphylococcus,* der ausgewählt ist aus der Gruppe bestehend aus *Staphylococcus vitulinus, Staphylococcus carnosus* und *Staphylococcus xylosus,* und gegebenenfalls mindestens eine Milchsäurebakterie.

4. Bakterielle Zusammensetzung nach einem der Ansprüche 1 bis 3, wobei der Stamm *Staphylococcus* ein *Staphylococcus vitulinus* ist, speziell der Stamm, der gemäß dem Budapester Vertrag vom 14. Januar 2014 beim Leibniz-Institut DSMZ unter der Nummer DSM28254 hinterlegt wurde.

5. Bakterielle Zusammensetzung nach einem der Ansprüche 1 bis 4, der gefroren, getrocknet, gefriergetrocknet, flüssig, fest, in der Form von Pellets oder gefrorenen Pellets oder als ein Pulver oder getrocknetes Pulver vorliegt.

6. Verfahren zu Herstellung eines vollständig gekochten NSS-Fleisches, umfassend:

    1) ein rohes Fleisch mit mindestens einer bakteriellen Zusammensetzung in Kontakt bringen, die mindestens einen Stamm *Streptococcus thermophilus* umfasst, wobei die bakterielle Zusammensetzung keine Lactose umfasst, um ein zubereitetes Fleisch zu erhalten; und
    2) Wärmebehandeln des zubereiteten Fleisches unter geeigneten Bedingungen, um ein vollständig gekochtes NSS-Fleisch zu erhalten, wobei die Wärmebehandlung das Kochen des zubereiteten Fleisches bis zu einer Kerntemperatur von mindestens 60 °C umfasst;

    wobei das Verfahren nicht einen fermentierenden Schritt umfasst.

7. Verfahren zu Herstellung eines vollständig gekochten NSS-Fleisches nach Anspruch 6, umfassend:

    1) ein rohes Fleisch mit mindestens einer bakteriellen Zusammensetzung in Kontakt bringen, die mindestens einen Stamm *Streptococcus thermophilus* umfasst, um ein zubereitetes Fleisch zu erhalten; und
    2a) Wärmebehandeln des zubereiteten Fleisches für mindestens 3 Stunden bis zu einer Kerntemperatur, die zwischen 20° und 50 °C liegt; und
    2b) Wärmebehandeln des Fleisches, das unter 2a) erhalten wurde, bis zu einer Kerntemperatur von mindestens 60 °C unter geeigneten Bedingungen, um ein vollständig gekochtes NSS-Fleisch zu erhalten.

8. Verfahren zur Herstellung eines vollständig gekochten NSS-Fleisches nach Anspruch 6 oder 7, wobei der mindestens eine Stamm *Streptococcus thermophilus* ein gering ansäuernder Stamm in Gegenwart von Kohlenhydrat(en) ist, speziell ein gering ansäuernder Stamm in Gegenwart von Saccharose ist und/oder in Salzlösung hoch resistent ist.

9. Verfahren nach einem der Ansprüche 6 bis 8, wobei die bakterielle Zusammensetzung wie in einem der Ansprüche 1 bis 5 festgelegt ist.

10. Verfahren nach einem der Ansprüche 6 bis 9, wobei das Fleisch bis zu einer Kerntemperatur von mindestens 65 °C, speziell mindestens 68 °C, speziell mindestens 70 °C gekocht wird.

11. Verfahren nach einem der Ansprüche 6 bis 10, wobei das rohe Fleisch mit der bakteriellen Zusammensetzung und mit einer Salzlösung in Kontakt gebracht wird, speziell mit der bakteriellen Zusammensetzung, die in einer Salzlösung enthalten ist.

**12.** Verfahren nach einem der Ansprüche 6 bis 11, wobei die bakterielle Zusammensetzung mit dem rohen Fleisch bei einer Konzentration von mindestens 1 x 10$^6$ KBE/g des zubereiteten Fleisches, speziell bei einer Konzentration von mindestens 5 x 10$^6$ KBE/g oder mindestens 1 x 10$^7$ KBE/g des zubereiteten Fleisches, in Kontakt gebracht wird.

**13.** Bakterielle Zusammensetzung nach einem der Ansprüche 1 bis 5, oder Verfahren nach einem der Ansprüche 6 bis 12, wobei der mindestens eine Stamm *Streptococcus thermophilus* ein gering ansäuernder Stamm ist, wie mit Hilfe von Assay III bestimmt wird und/oder ein für Salz toleranter Stamm ist, wie mit Hilfe von Assay II bestimmt wird.

**14.** Bakterielle Zusammensetzung nach einem der Ansprüche 1 bis 5 und Anspruch 13 oder Verfahren nach einem der Ansprüche 6 bis 13, wobei der Stamm *Streptococcus thermophilus* oder der mindestens eine Stamm *Streptococcus thermophilus* entweder einer von Folgendem ist oder ausgewählt ist aus der Gruppe bestehend aus dem Stamm, hinterlegt bei der DSMZ am 4. Dezember 2013 unter der Nummer DSM28128, dem Stamm, hinterlegt bei der DSMZ am 14. Januar 2014 unter der Nummer DSM28255, dem Stamm, hinterlegt bei der DSMZ am 14. Januar 2014 unter der Nummer DSM28256, dem Stamm, hinterlegt bei der DSMZ am 14. Januar 2014 unter der Nummer DSM28257, dem Stamm, hinterlegt bei der DSMZ am 14. Januar 2014 unter der Nummer DSM28258, und einer beliebigen Mischung davon.

**15.** Verfahren nach einem der Ansprüche 6 bis 14, wobei das Fleisch ein ganzes Muskelfleisch ist, insbesondere Schinken, oder Stücke von Fleisch ist.

**16.** Verwendung einer bakteriellen Zusammensetzung, umfassend mindestens einen Stamm *Streptococcus thermophilus,* wobei die bakterielle Zusammensetzung keine Lactose umfasst, um ein vollständig gekochtes NSS-Fleisch herzustellen, wobei die Verwendung keinen fermentierenden Schritt umfasst.

## Revendications

**1.** Composition bactérienne, appropriée pour la fabrication d'une viande non stable au stockage à température ambiante (NSS) totalement cuite, comprenant au moins une souche de *Streptococcus thermophilus* et au moins une souche bactérienne choisie dans le groupe constitué d'une souche de *Lactobacillus sakei,* d'une souche de *Lactobacillus curvatus*, d'une souche provenant du genre *Pediococcus* choisie dans le groupe constitué de *Pediococcus acidilactici* et *Pediococcus pentasaceus* et/ou d'une souche provenant du genre *Staphylococcus* choisie dans le groupe constitué de *Staphylococcus vitulinus*, *Staphylococcus carnosus* et *Staphylococcus xylosus.*

**2.** Composition bactérienne selon la revendication 1, dans laquelle ladite au moins une souche de *Streptococcus thermophilus* est une souche faiblement acidifiante en présence d'hydrate(s) de carbone, en particulier est une souche faiblement acidifiante en présence de saccharose, et/ou est hautement résistante dans une saumure.

**3.** Composition bactérienne selon la revendication 1 ou 2, comprenant au moins une souche de *Streptococcus thermophilus* et une souche de *Staphylococcus* choisie dans le groupe constitué de *Staphylococcus vitulinus*, *Staphylococcus carnosus* et *Staphylococcus xylosus* et optionnellement au moins une bactérie d'acide lactique.

**4.** Composition bactérienne selon l'une quelconque des revendications 1 à 3, dans laquelle ladite souche de *Staphylococcus* est une *Staphylococcus vitulinus*, en particulier la souche déposée sous le Traité de Budapest le 14 janvier 2014 au Leibniz-Institut DSMZ sous le numéro DSM28254.

**5.** Composition bactérienne selon l'une quelconque des revendications 1 à 4, qui est congelée, séchée, lyophilisée, liquide, solide, sous la forme de granulés ou de granulés congelés ou dans une poudre ou une poudre séchée.

**6.** Méthode pour fabriquer une viande NSS totalement cuite, comprenant:

1) la mise en contact d'une viande crue avec au moins une composition bactérienne comprenant au moins une souche de *Streptococcus thermophilus,* dans laquelle ladite composition bactérienne ne comprend pas de lactose, pour obtenir une viande préparée; et
2) le traitement thermique de ladite viande préparée dans des conditions appropriées pour obtenir une viande NSS totalement cuite, dans laquelle ledit traitement thermique comprend la cuisson de ladite viande préparée jusqu'à une température de cœur d'au moins 60°C;

où ladite méthode ne comprend pas d'étape de fermentation.

7. Méthode pour fabriquer une viande NSS totalement cuite selon la revendication 6, comprenant:

1) la mise en contact d'une viande crue avec au moins une composition bactérienne comprenant au moins une souche de *Streptococcus thermophilus,* pour obtenir une viande préparée;
2a) le traitement thermique de ladite viande préparée à une température de cœur comprise entre 20 et 50°C, pendant au moins 3 heures; et
2b) le traitement thermique de la viande obtenue dans l'étape 2a) jusqu'à une température de cœur d'au moins 60°C, dans des conditions appropriées, pour obtenir une viande NSS totalement cuite.

8. Méthode pour fabriquer une viande NSS totalement cuite selon la revendication 6 ou 7, dans laquelle ladite au moins une souche de *Streptococcus thermophilus* est une souche faiblement acidifiante en présence d'hydrate(s) de carbone, en particulier est une souche faiblement acidifiante en présence de saccharose, et/ou est hautement résistante dans une saumure.

9. Méthode selon l'une quelconque des revendications 6 à 8, dans laquelle ladite composition bactérienne est telle que définie dans l'une quelconque des revendications 1 à 5.

10. Méthode selon l'une quelconque des revendications 6 à 9, dans laquelle ladite viande est cuite jusqu'à une température de cœur d'au moins 65°C, en particulier au moins 68°C, en particulier au moins 70°C.

11. Méthode selon l'une quelconque des revendications 6 à 10, dans laquelle la viande crue est mise en contact avec ladite composition bactérienne et avec une saumure, en particulier avec ladite composition bactérienne contenue dans une saumure.

12. Méthode selon l'une quelconque des revendications 6 à 11, dans laquelle ladite composition bactérienne est mise en contact avec la viande crue à une concentration d'au moins $1 \times 10^6$ CFU/g de la viande préparée, en particulier à une concentration d'au moins $5 \times 10^6$ CFU/g ou d'au moins $1 \times 10^7$ CFU/g de la viande préparée.

13. Composition bactérienne selon l'une quelconque des revendications 1 à 5 ou méthode selon l'une quelconque des revendications 6 à 12, où ladite au moins une souche de *Streptococcus thermophilus* est une souche faiblement acidifiante comme il est déterminé par l'essai III et/ou est une souche tolérante au sel comme il est déterminé par l'essai II.

14. Composition bactérienne selon l'une quelconque des revendications 1 à 5 et 13 ou méthode selon l'une quelconque des revendications 6 à 13, où ladite souche de *Streptococcus thermophilus* ou ladite au moins une souche de *Streptococcus thermophilus* soit est, soit est choisie dans le groupe constitué de la souche déposée au DSMZ le 4 décembre 2013 sous le numéro DSM28128, la souche déposée au DSMZ le 14 janvier 2014 sous le numéro DSM28255, la souche déposée au DSMZ le 14 janvier 2014 sous le numéro DSM28256, la souche déposée au DSMZ le 14 janvier 2014 sous le numéro DSM28257, la souche déposée au DSMZ le 14 janvier 2014 sous le numéro DSM28258 et n'importe quel mélange de celles-ci.

15. Méthode selon l'une quelconque des revendications 6 à 14, dans laquelle ladite viande est une viande constituée entièrement de muscle, en particulier du jambon ou est des morceaux de viande.

16. Utilisation d'une composition bactérienne comprenant au moins une souche de *Streptococcus thermophilus*, où ladite composition bactérienne ne comprend pas de lactose, pour fabriquer une viande NSS totalement cuite, où ladite utilisation ne comprend pas d'étape de fermentation.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011025794 A, Bastian
- WO 9947007 A, Bailet
- WO 9728702 A
- WO 9921438 A

**Non-patent literature cited in the description**

- **BAWA et al.** *Journal of Food Science and Technology,* July 1985, vol. 22, 262-265
- Handbook of Meat and Meat Processing. 2012